Europäisches Patentamt
European Patent Office
Office européen des brevets

(19) (11) Numéro de publication: **0 376 824 A1**

(12) **DEMANDE DE BREVET EUROPEEN**

(21) Numéro de dépôt: **89403621.9**

(22) Date de dépôt: **22.12.89**

(51) Int. Cl.5: **C07D 405/04, C07D 213/64**

Revendications pour les Etats contractants suivants: ES + GR

(30) Priorité: **26.12.88 FR 8817194**

(43) Date de publication de la demande: **04.07.90 Bulletin 90/27**

(84) Etats contractants désignés: **AT BE CH DE ES FR GB GR IT LI LU NL SE**

(71) Demandeur: **SANOFI**
**40, Avenue George V**
**F-75008 Paris(FR)**

(72) Inventeur: **Garcia Georges**
**27, Rue des Aires**
**F-34980 Saint-Gely-Du-Fesc(FR)**
Inventeur: **Mettefeu, Daniel**
**285, Rue de Gênes**
**F-34000 Montpellier(FR)**
Inventeur: **Roux, Richard**
**9, Lotissement de L'Arnède**
**F-34570 Vailhauques(FR)**

(74) Mandataire: **Gillard, Marie-Louise et al**
**Cabinet Beau de Loménie 55, Rue d'Amsterdam**
**F-75008 Paris(FR)**

(54) **Procédé de préparaton de dérivés du chromanne et intermédiaires de synthèse.**

(57) L'invention décrit un procédé de synthèse d'un composé de formule :

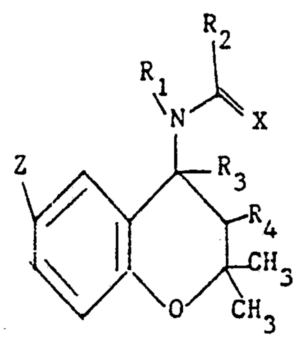

(I)

dans laquelle
- X = O ou NR,
- Z = groupement électro-attracteur,
- R = H, CN, $NO_2$,
- $R_1$ + $R_2$ + N-C=X = hétérocycle à 5 ou 6 chaînons,
- $R_3$ = H, $R_4$ = OH ou $R_3$ + $R_4$ = liaison,
procédé dans lequel des composés intermédiaires de formule :

EP 0 376 824 A1

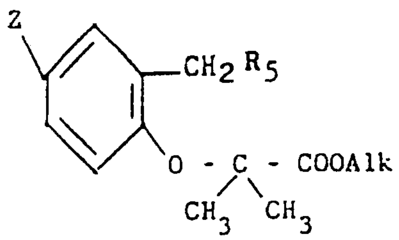

(IX)

dans laquelle $R_5$ = H, Br ou $N(R_1)CXR_2$ interviennent.
Ce procédé de synthèse ne fait pas intervenir de dérivé époxyde du chromanne.
Les composés nouveaux de formule (IX) constituent un autre objet de l'invention.

**Procédé de préparation de dérivés du chromanne et intermédiaires de synthèse.**

La présente invention a pour objet un nouveau procédé de synthèse de dérivés du chromanne. Elle concerne également de nouveaux composés, intermédiaires de cette synthèse.

Certains dérivés du chromanne ont été décrits pour leur activité antihypertensive, notamment dans J. Med. Chem., 1986, 29, 2194-2201.

Ces composés sont également décrits dans plusieurs demandes de brevets européens, par exemple les demandes : EP 76 075, EP 93 535, EP 273 262, EP 296 975, EP 312 432.

Plusieurs procédés de synthèse possibles sont décrits dans ces demandes de brevet. Ils font tous intervenir le dérivé époxyde du chromanne. Par exemple, on peut préparer un dérivé du chromannol par action d'un hétérocycle azoté sur l'époxyde du chromanne selon le schéma réactionnel suivant :

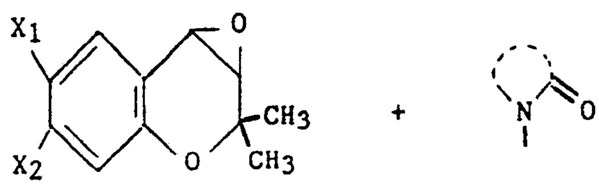

1

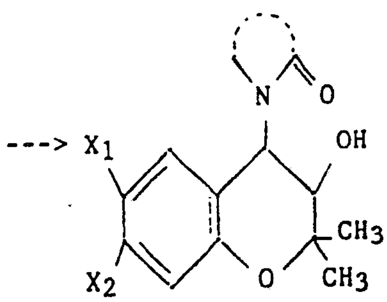

Dans les conditions opératoires décrites (demande de brevet EP 76075), le chromannol-3 obtenu a la configuration trans.

La préparation de l'époxyde 1 passe par la préparation intermédiaire d'un chromène de formule :

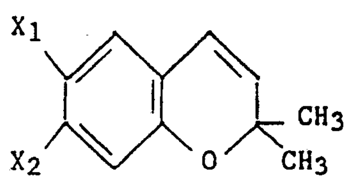

2

dont la distillation présente un danger d'explosion ou de décomposition.

Selon la présente invention, il a maintenant été trouvé un procédé de synthèse qui évite la préparation de l'époxyde 1 et du chromène 2.

La présente invention a pour objet un procédé pour la préparation d'un composé de formule :

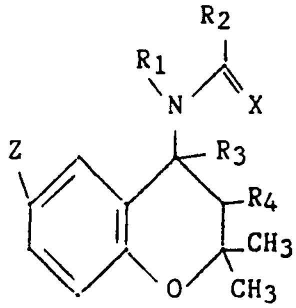

(I)

dans laquelle :
- X représente un atome d'oxygène ou un groupe NR,
- Z représente un groupement électro-attracteur,
- R représente l'hydrogène, un groupe cyano ou un groupe nitro,
- $R_1$ et $R_2$ forment avec le groupement -N-CX- auquel ils sont liés un hétérocycle à 5 ou 6 chaînons,
- $R_3$ représente l'hydrogène,
- $R_4$ représente un groupement hydroxyle,

ou $R_3$ et $R_4$ forment ensemble une liaison.

Les composés (I) dans lesquels le groupement Z est un atome d'halogéne, un groupe nitro ou un groupe cyano sont préférés.

Les composés (I) dans lesquels Z est un groupe cyano sont particulièrement préférés.

Les composés (I) dans lesquels le radical hétérocyclique constitué par le groupement -N($R_1$)-CO-$R_2$ représente la (dihydro-1,2 oxo-2) pyridyl-1 ou 1 oxo-2 pyrrolidinyl-1 sont particulièrement préférés.

Le procédé selon l'invention est caractérisé en ce que :

a) on éthérifie en milieu basique un méthyl-2 phénol substitué en position 4 par le groupement Z avec du bromo-2 méthyl-2 propionate d'éthyle ou du bromo-2 méthyl-2 propionate de méthyle, selon le schéma réactionnel suivant :

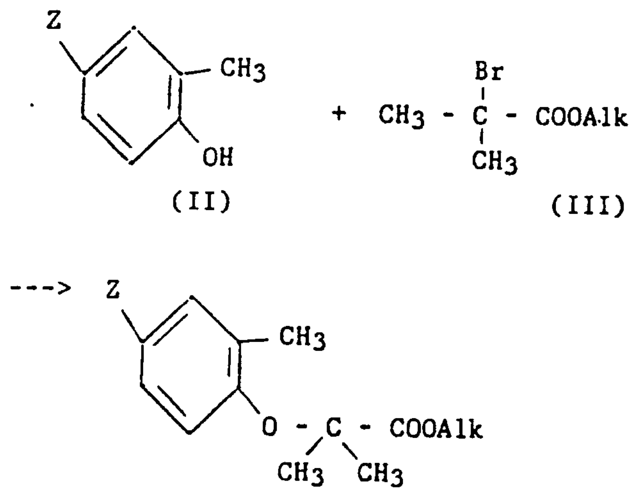

(II) + (III)

(IV)

Alk = $CH_3$ ou $CH_2CH_3$

b) éventuellement, on transforme le composé (IV) dans lequel Z = Br en un composé (IV) dans lequel Z = CN par action du cyanure cuivreux à chaud dans un solvant inerte en présence d'un catalyseur complexant du cuivre ;

c) on effectue la bromation du composé (IV) par le N-bromosuccinimide dans un solvant, en présence d'un catalyseur et en opérant sous irradiation par des rayons UV ;

d) on fait agir un hétérocycle azoté approprié de formule :

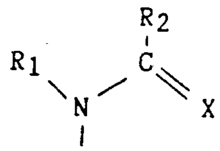

(V)

dans laquelle X, $R_1$ et $R_2$ ont les valeurs indiquées ci-dessus pour (I), sur le composé obtenu à l'étape c) de formule :

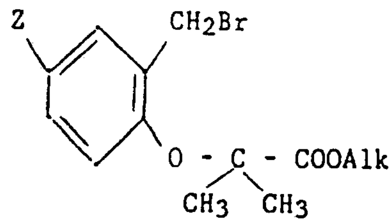

(VI),

la réaction est effectuée en présence d'un agent de condensation basique, dans un solvant inerte ;

e) on effectue la cyclisation du composé ainsi obtenu de formule:

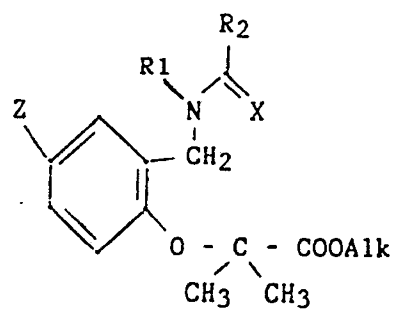

(VII)

par chauffage dans un solvant en milieu basique ;

f) on effectue la réduction du groupement oxo du composé ainsi obtenu de formule :

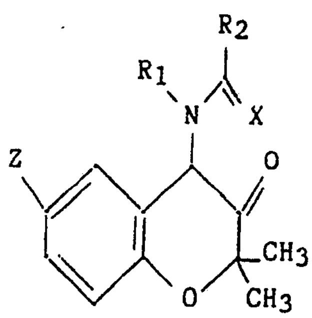

(VIII)

en présence d'un catalyseur tel qu'un borohydrure alcalin dans un solvant alcoolique pour obtenir un composé (I) dans lequel $R_3$ = H et $R_4$ = OH, sous forme d'un mélange cis et trans du chromannol-3;

g) éventuellement, si nécessaire, on sépare les isomères cis et trans du composé (I) précédemment obtenu par des procédés connus;

h) enfin, éventuellement, on déshydrate le composé obtenu à l'étape f) ou g) dans un solvant inerte en milieu alcalin à une température comprise entre 50 et 100 °C pour obtenir le composé (I) dans lequel $R_3$ et $R_4$ forment une liaison.

A l'étape a), la réaction est effectuée à chaud, de préférence en présence de carbonate de potassium ou de potasse, éventuellement dans un solvant, par exemple le méthanol ou l'éthanol et, éventuellement, en

présence d'un catalyseur tel qu'un catalyseur de transfert de phase comme l'hydroxyde de triméthyl benzyl ammonium.

A l'étape b), la substitution éventuelle du brome par le cyano est effectuée en choisissant comme catalyseur de préférence le tris dioxa-3,6 heptylamine et dans un solvant tel que le diméthylformamide.

Selon l'invention, on peut donc obtenir le composé (IV) dans lequel Z = CN par 2 voies de synthèse différentes :

- soit directement à partir du composé (II) dans lequel Z = CN, selon l'étape a), - soit en 2 étapes : a) + b) à partir du composé (II) dans lequel Z = Br.

A l'étape c), le catalyseur préféré pour la bromation est le péroxyde de benzoyle utilisé dans un solvant tel que, par exemple, le tétrachlorure de carbone.

A l'étape d), on peut utiliser comme agent de condensation un hydrure métallique tel que, par exemple, l'hydrure de sodium, la réaction est préférentiellement réalisée dans le diméthylformamide.

A l'étape e), la cyclisation du composé (VII) peut être effectuée en présence d'un hydrure métallique, ou, préférentiellement, en présence d'un alcoolate métallique tel que, par exemple, le tertiobutylate de potassium, dans un solvant tel que le tétrahydrofuranne ou le dioxanne.

A l'étape f), on peut effectuer la réduction du groupement oxo du composé (VIII) en présence de borohydrure de sodium dans le méthanol ou l'éthanol.

A l'étape h), la déshydratation est effectuée préférentiellement à chaud, en présence d'un hydrure alcalin tel que par exemple l'hydrure de sodium dans un solvant comme le tétrahydrofuranne ou le dioxanne.

Préférentiellement, le procédé selon l'invention est utilisé pour préparer le cyano-6 (dihydro-1,2 oxo-2 pyridyl-1)-4 diméthyl-2,2 hydroxy-3 chromanne, sous forme cis et trans et le cyano-6 (dihydro-1,2 oxo-2 pyridyl-1)-4 diméthyl-2,2 chromène. Dans ce cas l'hétérocycle azoté (V) ajouté à l'étape 4 est l'hydroxy-2 pyridine.

Les produits de départ sont connus ou obtenus par des méthodes connues.

Ainsi le nitro-4 méthyl-2 phénol est connu et préparé à partir de l'aniline correspondante qui est commerciale.

Le bromo-4 méthyl-2 phénol est décrit dans J. Pract. Chem. 1888, [2] 38, 324, il est préparé à partir de la bromo-4 méthyl-2 aniline.

Le cyano-4 méthyl-2 phénol est préparé à partir du bromo-4 méthyl-2 phénol selon la méthode décrite dans J. Org. Chem., 1957, 1669.

Un article paru dans Acta Pharm. Suec., 1976, 13, 427-438, décrit certains dérivés de l'acide phénoxy-2 méthyl-2 propionique de formule :

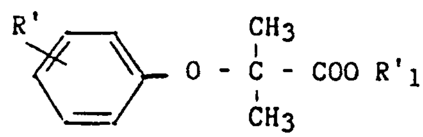

Seulement deux parmi les composés décrits portent un autre substituant $R''$ sur le noyau benzénique et en particulier un composé 3 dans lequel $R' = Cl$-4, $R'' = CH_3$-2 et $R'_1 = C_2H_5$. Ces composés sont des agents potentiellement hypolipémiants.

Les composés de formule :

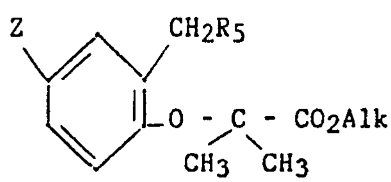

(IX)

dans laquelle Z et Alk ont les significations indiquées ci-dessus et $R_5$ représente l'hydrogène, un atome de brome ou un groupement $N(R_1)CXR_2$, X ayant les significations indiquées ci-dessus, sont nouveaux à la condition que Z soit différent d'un atome de chlore lorsque $R_5$ représente l'hydrogène et Alk un groupe éthyle. Ces nouveaux composés représentent un aspect ultérieur de la présente invention.

L'exemple suivant illustre l'invention. Les composés obtenus sont caractérisés par leur point de fusion (Fc), leur point d'ébullition (Eb) ou de leur spectre infra-rouge (IR). De plus, les spectres RMN ont été

enregistrés et ils sont en conformité avec les structures des composés décrits.

EXEMPLE :

Cyano-6 (dihydro-1,2 oxo-2 pyridyl-1)-4 diméthyl-2,2 chromène.

A) (bromo-4 méthyl-2 phénoxy)-2 méthyl-2 propionate d'éthyle.

On porte à 120° C un mélange contenant 105,6 g de bromo-4 méthyl-2 phénol et 220,4 g de bromo-2 méthyl-2 propionate d'éthyle, on introduit lentement 78,1 g de carbonate de potassium puis on chauffe à 110° C pendant 5 heures. On ramène la température à 100° C puis on dilue le milieu réactionnel par 1,5 litre d'éthanol aqueux, puis, à 80° C, on acidifie à pH = 2 par de l'acide sulfurique concentré. Après concentration sous vide du solvant, on reprend le résidu par de l'eau et éther éthylique. On observe la formation d'un gel. Après décantation, la phase organique est lavée par une solution à 4 % de soude puis à l'eau. On sèche sur sulfate de sodium puis concentre le solvant sous vide.

L'huile orange récupérée (150 g) est distillée sous vide. On obtient 120 g d'huile limpide.

Eb : 120-130° C sous 20 Pa (0,15 mm Hg)
IR : 1730 $cm^{-1}$
Rf ($CH_2Cl_2$/hexane : 5/5) : 0,46
Rendement : 71 %.

B) (Cyano-4 méthyl-2 phénoxy)-2 méthyl-2 propionate d'éthyle.

On chauffe à reflux pendant 12 heures un mélange contenant 29 g du composé préparé à l'étape précédente, 15 g de cyanure cuivreux dans 50 ml de tris dioxa-3,6 heptylamine et 100 ml de diméthylformamide. On refroidit puis on verse sur un mélange d'un litre d'eau et 500 ml d'acétate d'éthyle. On laisse une demi-heure sous agitation puis on filtre sur silice diatomée (Célite®). Après décantation, on lave à l'eau la phase organique (2 fois), sèche sur sulfate de sodium et concentre. L'huile obtenue est filtrée sur une colonne de silice en éluant par de l'éther isopropylique pour éliminer les sels de cuivre. Par cristallisation dans l'hexane on obtient 18,5 g du produit attendu.

Fc : 46° C
IR : 2230 $cm^{-1}$
Rf (hexane/acétone : 70/30) : 0,38
Rendement : 77 %.

Un procédé alternatif pour préparer ce composé est décrit ci-dessous

On chauffe à reflux pendant 8 heures un mélange contenant 5 g de cyano-4 méthyl-2 phénol, 2,8 g de potasse, 14,5 g de bromo-2 méthyl-2 propionate d'éthyle, 50 ml d'éthanol et 0,5 ml d'Aliquot 336®, un ammonium quaternaire commercialisé par ALDRICH. On concentre le solvant puis on reprend par 100 ml d'eau. On extrait par l'éther éthylique, on lave la phase organique par une solution de soude IN puis par de l'eau. On sèche sur sulfate de sodium et concentre. On purifie ensuite le produit obtenu par chromatographie sur colonne de silice en éluant par le pentane. On recueille 4,3 g du composé attendu.

Fc = 44° C
Rendement : 60 %.

C) (Bromométhyl-2 cyano-4 phénoxy)-2 méthyl-2 propionate d'éthyle.

On chauffe à reflux pendant 3 heures, sous irradiation par des rayons UV, un mélange contenant 15 g du composé obtenu à l'étape précédente, 13,5 g de N-bromo succinimide dans 200 ml de tétrachlorure de carbone et 20 mg de péroxyde de benzoyle. On refroidit, puis on lave à l'eau (2 fois), sèche sur sulfate de sodium et concentre. On recristallise dans un mélange éther isopropylique-hexane (50 - 100) et l'on obtient 15 g du produit attendu.

Fc = 72° C
Rf (hexane/acétone : 70/30) : 0,31

Rendement : 75,8 %

D) Cyano-4 [(dihydro-1,2 oxo-2 pyridyl-1) méthyl]-2 phénoxy-2 méthyl-2 propionate d'éthyle.

A une solution de 3 g d'hydroxy-2 pyridine dans 50 ml de diméthylformamide, on ajoute par petites fractions 900 mg d'hydrure de sodium puis on laisse sous agitation pendant 30 minutes à température ambiante. On ajoute 9 g du composé obtenu à l'étape précédente puis on laisse sous agitation 16 heures à température ambiante puis 2 heures à 60 °C. On concentre la diméthylformamide puis le résidu est repris dans l'eau et extrait à l'éther éthylique (2 fois). La phase organique est lavée à l'eau puis séchée sur sulfate de sodium. L'huile obtenue (9 g) est purifiée par chromatographie sur colonne de silice en éluant par de l'acétate d'éthyle. On recueille 6,5 g du produit attendu
Fc : 73,1 °C
Rf (acétate d'éthyle) : 0,28
Rendement : 69,1 %.

E) Cyano-6 (dihydro-1,2 oxo-2 pyridyl-1)-4 diméthyl-2,2 oxo-3 chromanne.

On ajoute par petites fractions 750 mg de tertiobutylate de potassium à une solution de 2 g, du composé obtenu à l'étape précédente dans 50 ml de tétrahydrofuranne. On laisse 4 heures sous agitation à température ambiante. On concentre le solvant, reprend le résidu par 50 ml d'eau puis acidifie par de l'acide chlorhydrique concentré. On extrait par de l'éther éthylique, lave à l'eau, sèche sur sulfate de sodium et concentre. L'huile obtenue est purifiée par chromatographie sur colonne de silice en éluant par un mélange $CH_2Cl_2/CH_3OH$ : 99/1. On recristallise dans l'éther isopropylique pour obtenir 950 mg du produit attendu
Fc : 178 °C
Rendement : 56 %.

F) cis et trans Cyano-6 (dihydro-1,2 oxo-2 pyridyl-1)-4 diméthyl-2,2 hydroxy-3 chromanne.

On mélange 1,7 g du composé obtenu à l'étape précédente et 45 ml de méthanol et l'on ajoute à température ambiante 0,114 g de borohydrure de sodium puis on laisse 3 heures sous agitation à température ambiante. L'excès de réactif est détruit par addition d'acide acétique puis on concentre le solvant sous vide. Le résidu est repris par de l'eau puis extrait à l'acétate d'éthyle. La phase organique est lavée à l'eau, séchée sur sulfate de sodium puis concentrée sous vide. On récupère le produit attendu sous forme d'une mousse solide blanche utilisée telle quelle à l'étape suivante :
Rf ($CH_2Cl_2/CH_3OH$ : 95/5) : 0,34
Rendement : 82 %.

G) Cyano-6 (dihydro-1,2 oxo-2 pyridyl-1)-4 diméthyl-2,2 chromène.

1,4 g du composé obtenu à l'étape précédente sont placés dans 44 ml de tétrahydrofuranne anhydre et l'on ajoute par portions, à température ambiante, 0,132 g d'hydrure de sodium à 50 % dans l'huile puis on chauffe à reflux pendant 5 heures. On concentre le solvant sous vide, reprend le résidu par du dichlorométhane puis lave la phase organique à l'eau jusqu 'à neutralité et sèche sur sulfate de sodium.

Après concentration sous vide, on récupère 1 g du composé attendu sous forme d'un solide blanc.
Fc : 151 °C
Rendement : 73 %.

## Revendications

1. Procédé pour la préparation d'un composé de formule :

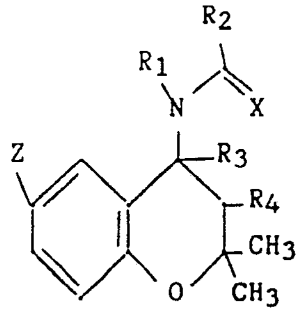

(I)

dans laquelle :

- X représente l'oxygène ou un groupe N = R,
- Z représente un groupement électro-attracteur,
- R représente un atome d'hydrogène, un groupe nitro ou un groupe cyano,
- $R_1$ et $R_2$ forment avec le groupement -N-CX- auquel ils sont liés un hétérocycle à 5 ou 6 chaînons,
- $R_3$ représente l'hydrogène,
- $R_4$ représente un groupement hydroxyle,

ou $R_3$ et $R_4$ forment ensemble une liaison,

caractérisé en ce que :

a) on éthérifie en milieu basique un méthyl-2 phénol substitué en position 4 par le groupement Z avec du bromo-2 méthyl-2 propionate d'éthyle ou du bromo-2 méthyl-2 propionate de méthyle, selon le schéma réactionnel suivant :

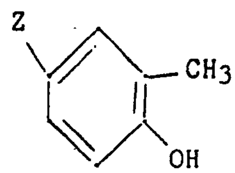

(II)

+

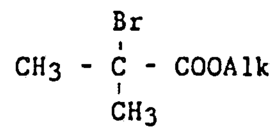

(III)

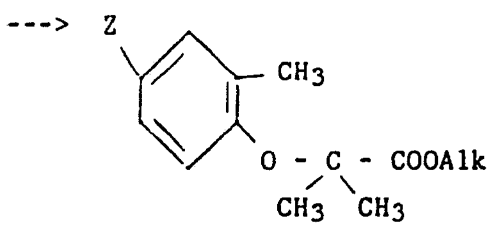

(IV)

Alk = $CH_3$ ou $CH_2CH_3$

b) éventuellement, on transforme le composé (IV) dans lequel Z = Br en un composé (IV) dans lequel Z = CN par action du cyanure cuivreux à chaud dans un solvant inerte en présence d'un catalyseur complexant du cuivre ;

c) on effectue la bromation du composé (IV) par le N-bromosuccinimide dans un solvant, en présence d'un catalyseur et en opérant sous irradiation par des rayons UV ;

d) on fait agir un hétérocycle azoté approprié de formule :

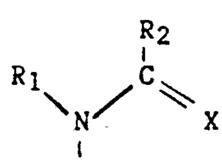

(V)

dans laquelle X, $R_1$ et $R_2$ ont les valeurs indiquées ci-dessus pour (I), sur le composé obtenu à l'étape c) de formule :

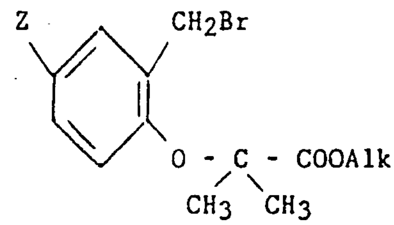

(VI),

la réaction est effectuée en présence d'un agent de condensation basique, dans un solvant inerte ;

e) on effectue la cyclisation du composé ainsi obtenu de formule:

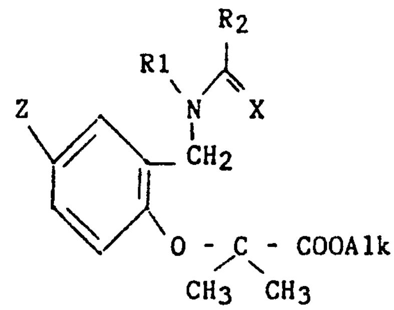

(VII)

par chauffage dans un solvant en milieu basique ;

f) on effectue la réduction du groupement oxo du composé ainsi obtenu de formule :

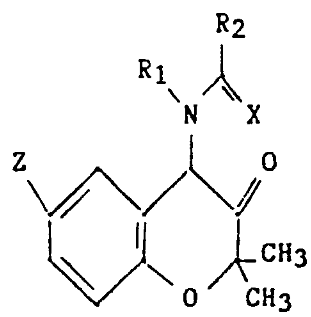

(VIII)

en présence d'un catalyseur tel qu'un borohydrure alcalin dans un solvant alcoolique pour obtenir un composé (I) dans lequel $R_3$ = H et $R_4$ = OH, sous forme d'un mélange cis et trans du chromannol-3;

g) éventuellement, si nécessaire, on sépare les isomères cis et trans du composé (I) précédemment obtenu par des procédés connus;

h) enfin, éventuellement, on déshydrate le composé obtenu à l'étape f) ou g) dans un solvant inerte en milieu alcalin à une température comprise entre 50 et 100 °C pour obtenir le composé (I) dans lequel $R_3$ et $R_4$ forment une liaison.

2. Procédé selon la revendication 1, caractérisé en ce que l'étape a) est effectuée à chaud, de préférence en présence de carbonate de potassium ou de potasse, éventuellement dans un solvant, par exemple le méthanol ou l'éthanol et, éventuellement, en présence d'un catalyseur tel qu'un catalyseur de transfert de phase comme l'hydroxyde de triméthyl benzyl ammonium.

3. Procédé selon l'une des revendications 1 ou 2, caractérisé en ce que la substitution du brome par le groupement cyano, à l'étape b), est effectuée en présence de tris dioxa-3,6 heptylamine dans un solvant tel que le diméthylformamide.

4. Procédé selon l'une quelconque des revendications 1 à 3, caractérisé en ce que la bromation du composé (IV), à l'étape c) est effectuée dans un solvant tel que le tétrachlorure de carbone en présence de

péroxyde de benzoyle.

5. Procédé selon l'une quelconque des revendications 1 à 4, caractérisé en ce que l'étape d) est réalisée préférentiellement dans le diméthylformamide, en présence d'un hydrure métallique, tel que l'hydrure de sodium.

6. Procédé selon l'une quelconque des revendications 1 à 5, caractérisé en ce que la cyclisation du composé (VII), à l'étape e) est effectuée en présence d'un hydrure métallique, ou préférentiellement, en présence d'un alcoolate métallique tel que, par exemple, le tertiobutylate de potassium, dans un solvant tel que le tétrahydrofuranne ou le dioxanne.

7. Procédé selon l'une quelconque des revendications 1 à 6, caractérisé en ce que la réduction du composé (VIII), à l'étape f), est effectuée en présence de borohydrure de sodium dans le méthanol ou l'éthanol.

8. Procédé selon l'une quelconque des revendications 1 à 7, caractérisé en ce que la déshydratation, à l'étape h), est effectuée préférentiellement à chaud, en présence d'un hydrure alcalin, tel que par exemple l'hydrure de sodium, dans un solvant comme le tétrahydrofuranne ou le dioxanne.

9. Procédé selon l'une quelconque des revendications précédentes, caractérisé en ce qu'il est mis en oeuvre, à l'étape a), avec un composé (II) dans lequel le groupement Z est un atome d'halogène, un groupe nitro ou un groupe cyano.

10. Procédé selon l'une quelconque des revendications précédentes, caractérisé en ce qu'il est mis en oeuvre, à l'étape d), avec l'hydroxy-2 pyridine ou l'oxo-2 pyrrolidine comme hétérocycle azoté (V).

11. Procédé selon l'une quelconque des revendications 1 à 8, pour la préparation du cyano-6 (dihydro-1,2 oxo-2 pyridyl-1)-4 diméthyl-2,2 hydroxy-3 chromanne, sous forme cis ou trans et du cyano-6 (dihydro-1,2 oxo-2 pyridyl-1)-4 diméthyl-2,2 chromène, caractérisé en ce qu'il est mis en oeuvre, à l'étape a), avec un composé (II) dans lequel le groupement Z représente un atome de brome ou un groupe cyano et, à l'étape d), avec l'hydroxy-2 pyridine comme hétérocycle azoté (V).

12. Intermédiaires de synthèse pour la préparation des composés (I) selon la revendication 1, caractérisés en qu'ils répondent à la formule :

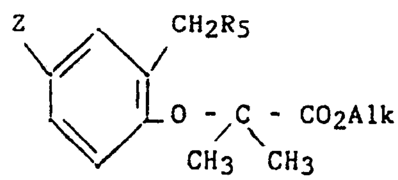

(IX)

dans laquelle

- Z représente un groupement électro-attracteur,
- Alk représente un méthyle ou un éthyle,
- $R_5$ représente l'hydrogène, un atome de brome ou un groupement $N(R_1)CXR_2$ ; $R_1$, $R_2$ et X étant tels que définis à la revendication 1 ;à la condition que Z soit différent d'un atome de chlore lorsque $R_5$ représente l'hydrogène et Alk un groupe éthyle.

13. Composé de formule (IX) selon la revendication 12, dans laquelle Z représente un groupe cyano, $R_5$ représente l'hydrogène, un atome de brome ou un groupement (dihydro-1,2 oxo-2 pyridyl-1).

Revendications pour les Etats contractants suivants: ES, GR.

1. Procédé pour la préparation d'un composé de formule :

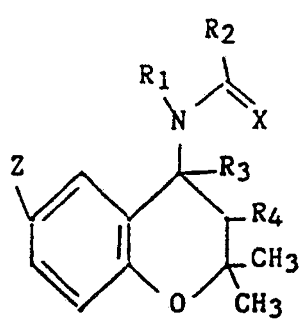

(I)

dans laquelle :
- X représente l'oxygène ou un groupe N = R,
- Z représente un groupement électro-attracteur,
- R représente un atome d'hydrogène, un groupe nitro ou un groupe cyano,
- $R_1$ et $R_2$ forment avec le groupement -N-CX- auquel ils sont liés un hétérocycle à 5 ou 6 chaînons,
- $R_3$ représente l'hydrogène,
- $R_4$ représente un groupement hydroxyle,
ou $R_3$ et $R_4$ forment ensemble une liaison,
caractérisé en ce que :

a) on éthérifie en milieu basique un méthyl-2 phénol substitué en position 4 par le groupement Z avec du bromo-2 méthyl-2 propionate d'éthyle ou du bromo-2 méthyl-2 propionate de méthyle, selon le schéma réactionnel suivant :

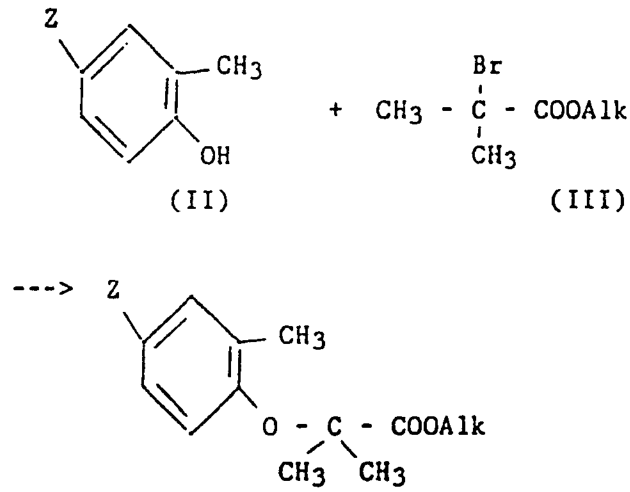

(II) (III)

(IV)

Alk = $CH_3$ ou $CH_2CH_3$

b) éventuellement, on transforme le composé (IV) dans lequel Z = Br en un composé (IV) dans lequel Z = CN par action du cyanure cuivreux à chaud dans un solvant inerte en présence d'un catalyseur complexant du cuivre ;

c) on effectue la bromation du composé (IV) par le N-bromosuccinimide dans un solvant, en présence d'un catalyseur et en opérant sous irradiation par des rayons UV ;

d) on fait agir un hétérocycle azoté approprié de formule :

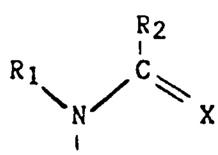 (V)

dans laquelle X, $R_1$ et $R_2$ ont les valeurs indiquées ci-dessus pour (I), sur le composé obtenu à l'étape c) de formule :

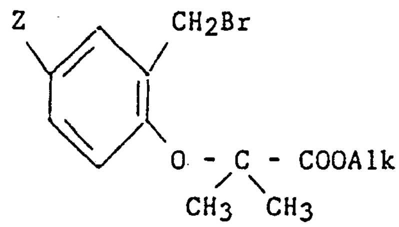 (VI),

la réaction est effectuée en présence d'un agent de condensation basique, dans un solvant inerte ;

e) on effectue la cyclisation du composé ainsi obtenu de formule:

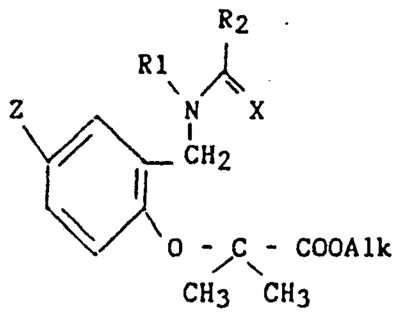 (VII)

par chauffage dans un solvant en milieu basique ;

f) on effectue la réduction du groupement oxo du composé ainsi obtenu de formule :

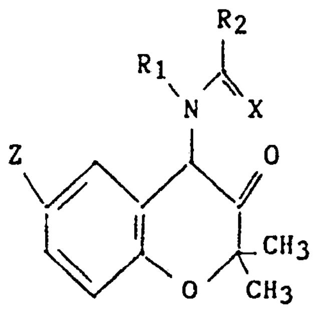 (VIII)

en présence d'un catalyseur tel qu'un borohydrure alcalin dans un solvant alcoolique pour obtenir un composé (I) dans lequel $R_3$ = H et $R_4$ = OH, sous forme d'un mélange cis et trans du chromannol-3;

g) éventuellement, si nécessaire, on sépare les isomères cis et trans du composé (I) précédemment obtenu par des procédés connus;

h) enfin, éventuellement, on déshydrate le composé obtenu à l'étape f) ou g) dans un solvant inerte en milieu alcalin à une température comprise entre 50 et 100 °C pour obtenir le composé (I) dans lequel $R_3$ et $R_4$ forment une liaison.

2. Procédé selon la revendication 1, caractérisé en ce que l'étape a) est effectuée à chaud, de préférence en présence de carbonate de potassium ou de potasse, éventuellement dans un solvant, par exemple le méthanol ou l'éthanol et, éventuellement, en présence d'un catalyseur tel qu'un catalyseur de transfert de phase comme l'hydroxyde de triméthyl benzyl ammonium.

3. Procédé selon l'une des revendications 1 ou 2, caractérisé en ce que la substitution du brome par le groupement cyano, à l'étape b), est effectuée en présence de tris dioxa-3,6 heptylamine dans un solvant tel que le diméthylformamide.

4. Procédé selon l'une quelconque des revendications 1 à 3, caractérisé en ce que la bromation du composé (IV), à l'étape c) est effectuée dans un solvant tel que le tétrachlorure de carbone en présence de péroxyde de benzoyle.

5. Procédé selon l'une quelconque des revendications 1 à 4, caractérisé en ce que l'étape d) est réalisée préférentiellement dans le diméthylformamide, en présence d'un hydrure métallique, tel que

l'hydrure de sodium.

6. Procédé selon l'une quelconque des revendications 1 à 5, caractérisé en ce que la cyclisation du composé (VII), à l'étape e) est effectuée en présence d'un hydrure métallique, ou préférentiellement, en présence d'un alcoolate métallique tel que, par exemple, le tertiobutylate de potassium, dans un solvant tel que le tétrahydrofuranne ou le dioxanne.

7. Procédé selon l'une quelconque des revendications 1 à 6, caractérisé en ce que la réduction du composé (VIII), à l'étape f), est effectuée en présence de borohydrure de sodium dans le méthanol ou l'éthanol.

8. Procédé selon l'une quelconque des revendications 1 à 7, caractérisé en ce que la déshydratation, à l'étape h), est effectuée préférentiellement à chaud, en présence d'un hydrure alcalin, tel que par exemple l'hydrure de sodium, dans un solvant comme le tétrahydrofuranne ou le dioxanne.

9. Procédé selon l'une quelconque des revendications précédentes, caractérisé en ce qu'il est mis en oeuvre, à l'étape a), avec un composé (II) dans lequel le groupement Z est un atome d'halogène, un groupe nitro ou un groupe cyano.

10. Procédé selon l'une quelconque des revendications précédentes, caractérisé en ce qu'il est mis en oeuvre, à l'étape d), avec l'hydroxy-2 pyridine ou l'oxo-2 pyrrolidine comme hétérocycle azoté (V).

11. Procédé selon l'une quelconque des revendications 1 à 8, pour la préparation du cyano-6 (dihydro-1,2 oxo-2 pyridyl-1)-4 diméthyl-2,2 hydroxy-3 chromanne, sous forme cis ou trans et du cyano-6 (dihydro-1,2 oxo-2 pyridyl-1)-4 diméthyl-2,2 chromène, caractérisé en ce qu'il est mis en oeuvre, à l'étape a), avec un composé (II) dans lequel le groupement Z représente un atome de brome ou un groupe cyano et, à l'étape d), avec l'hydroxy-2 pyridine comme hétérocycle azoté (V).

12. Procédé pour la préparation d'intermédiaires de synthèse de formule :

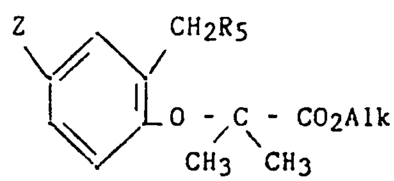

(IX)

dans laquelle

- Z représente un groupement électro-attracteur,
- Alk représente un méthyle ou un éthyle,
- $R_5$ représente l'hydrogène, un atome de brome ou un groupement $N(R_1)CXR_2$ ; $R_1$, $R_2$ et X étant tels que définis à la revendication 1 ;à la condition que Z soit différent d'un atome de chlore lorsque $R_5$ représente l'hydrogène et Alk un groupe éthyle ;

utiles à la préparation des composés (I) selon la revendication 1, caractérisé en ce qu'il consiste à mettre en oeuvre

- les étapes a) et éventuellement b) du procédé selon la revendication 1 pour la préparation des composés de formule (IX) dans laquelle $R_5$ représente l'hydrogène ;
- les étapes a), éventuellement b) et c) du procédé selon la revendication 1 pour la préparation des composés de formule (IX) dans laquelle $R_5$ représente le brome ;
- les étapes a), éventuellement b), c) et d) du procédé selon la revendication 1 pour la préparation des composés de formule (IX) dans laquelle $R_5$ représente le groupement $N(R_1)CXR_2$ ;

à partir des composés appropriés de formule (II), (III) et éventuellement (IV).

13. Procédé selon la revendication 12 pour la préparation d'intermédiaires de formule (IX) dans laquelle Z représente un groupe cyano, $R_5$ représente l'hydrogène, un atome de brome ou un groupement (dihydro-1,2 oxo-2 pyridyl-1) caractérisé en ce qu'il est mis en oeuvre à partir des composés de formule (II), dans laquelle Z = CN ou Br, de formule (III) et éventuellement de l'hydroxy-2 pyridine de formule (V).

Office européen des brevets

# RAPPORT DE RECHERCHE EUROPEENNE

Numero de la demande

EP 89 40 3621

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 86, 1977, page 15, résumé no. 150317y, Columbus, Ohio, US; T. HOGBERG et al.: "Potential hypolipidemic agents. XV. Synthesis and plasma lipid-lowering properties of derivatives of 2-phenoxy-2-methylpropionic acid", & ACTA PHARM. SUEC. 1976, 13(5-6), 427-38 * Résumé * --- | 12 | C 07 D 405/04<br>C 07 D 213/64 |
| D,A | EP-A-0 273 262 (MERCK) * Pages 1,5,6 * ----- | 1 | |
| | | | **DOMAINES TECHNIQUES RECHERCHES (Int. Cl.5)** |
| | | | C 07 D 405/00<br>C 07 D 213/00 |

Le présent rapport a été établi pour toutes les revendications

| Lieu de la recherche | Date d'achèvement de la recherche | Examinateur |
|---|---|---|
| LA HAYE | 21-02-1990 | FRANCOIS J.C.L. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant